# EUROPEAN PATENT SPECIFICATION

(11) **EP 0 770 590 B1**
(45) Date of publication and mention of the grant of the patent: **24.10.2001**
(21) Application number: 96306992.7
(22) Date of filing: 25.09.1996
(51) Int. Cl.: C07C 37/20, C07C 39/16, B01J 31/08

(54) **Process for the preparation of bisphenol compounds with simultaneous dehydration**
Verfahren zur Herstellung von Bisphenol-Verbindungen mit gleichzeitiger Entwässerung
Procédé de préparation de composés bisphénols avec déshydratation simultanée

(30) Priority: 29.09.1995 US 536508; 09.08.1996 US 690484
(43) Date of publication of application: 02.05.1997
(73) Proprietor: GENERAL ELECTRIC COMPANY, Schenectady, NY 12345 (US)
(72) Inventor: Kissinger, Gaylord Michael, Evansville, Indiana 47711 (US); Sybert, Paul Dean, Evansville, Indiana 47712 (US)
(74) Representative: Szary, Anne Catherine, Dr.

(56) References cited:
- EP-A- 0 442 122
- WO-A-94/19302
- US-A- 3 634 341
- PATENT ABSTRACTS OF JAPAN vol. 010, no. 252 (C-369), 29 August 1986 & JP-A-61 078741 (MITSUI TOATSU CHEM INC), 22 April 1986,
- DATABASE WPI Week 7747 Derwent Publications Ltd., London, GB; AN 77-83879Y XP002020834 "Antioxidant (2,2')-methylene-bis-dialkylphenol prepn. - by reacting dialkyl phenol with para-formaldehyde in inert solvent" & JP-A-52 122 350 (SEIKO KAGAKU KK) , 14 October 1977

## Description

The invention relates to processes and apparatus for condensation of a bisphenol with a ketone, in the presence of acidic ion-exchange resins as catalysts.

### Brief Description of Related Art

The preparation of bisphenols by the condensation of monocyclic phenols with a ketone in the presence of an acid catalyst is a widely known procedure; see for example the description in U.S. Patent 2,858,342. As recognized in this early patent, the condensation reaction forms one mole of water co-product for each mole of reactants condensed. The presence of this water co-product during subsequent reaction of the phenols with the ketones, acts to lower the reflux temperatures of the reaction mixture. Consequently, in the U.S. Patent 2,858,342 it is suggested that the water of condensation be removed during the reaction by distillation into a Dean-Stark trap.

Later processes employ as the condensation catalyst acidic ion-exchange resins; see for example the descriptions found in U.S. Patents 3,634,341 and 3,760,006. These descriptions point out that water content of the resin catalyst is an important process variable. Catalyst efficiency is impaired if the water content of the reaction mixture is more than about 2-3 weight percent, especially in a fixed bed reactor. In the U.S. Patent 3,634,341 it is said that the resin catalyst should be dried, if necessary, by passing anhydrous phenol through the bed of resin.

A catalyst utilized in the commercial processes is a macro-reticular or gelular, acidic ion-exchange resin; see for example the U.S. Patent 4,191,843 (Kwantes et al). These catalysts are the product of monoethylenically unsaturated monomers copolymerized with polyvinylidene monomers, as described for example in the U.S. Patent 3,037,052 (Bortnick). Preferred macro-reticular resins are sulfonated to position sulfonic acid groups, pendant from the aromatic nucleus of the vinylidene moiety; see the U.S. Patent 3,037,052.

The above-described acid catalyst (sulfocationites) contains up to 40 to 85 percent by weight of water and are generally made available by the manufacturer in this hydrated state. The presence of the water will inhibit the desired catalyst activity in condensing phenols with ketones, because the water will bind to the sulfonic acid groups in competition with reactant. For this reason, Bortnick (U.S. Patent 3,037,052) teaches one to dehydrate the catalyst prior to use. As a method of dehydration, Bortnick suggests "drying at 105°C. to about 125°C. at a pressure of 5 to 10 mm" or by azeotropic distillation with an organic liquid (column 4, lines 54-64).

Kwantes et al., in U.S. Patent 4,191,843 describing in particular the preparation of bisphenols, states that "the reactor may be filled with the acid ion exchanger by any known technique. Such techniques include adding the desired amount of dry ion exchanger, water wet ion exchanger or slurry of the ion exchanger in the reactor"; see column 2, lines 51-55. Details of the drying procedure are not elaborated upon beyond stating that the reactor charged resin is "drained"; see the patent Example 1.

In the commercially practiced processes for preparing bisphenol-A, acid ion-exchange resins are dried in the plant reactor (usually a column reactor) prior to initiation of the reaction between phenol and acetone. Drying is effected by passing phenol over the catalyst in the absence of acetone, to absorb and/or displace the water associated with the acid ion-exchange resin. Water that is absorbed or displaced by the phenol is removed from the resin bed as an effluent stream. The water is ultimately removed from the phenol effluent by employing conventional separation techniques such as distillation and extraction.

The aforementioned dehydration procedure is time consuming, often requiring several weeks and results in substantial reactor down time. Where a plant utilizes large amounts of catalyst resin, resin addition and drying can take several months. Furthermore, cycles of resin addition and dehydration are known to stress and damage macro-reticular or gelular resin beads. This deleteriously affects reaction mixture flow in the reactor and pressure drop across the bed.

One solution considered for the problems associated with the commercially practiced procedure of drying the acidic ion-exchange resin is to follow the teachings of the aforementioned U.S. Patent 3,037,052 and pre-dry the resin to an anhydrous state before it is charged to the column reactor. However, this procedure also has certain disadvantages. For example, if the resin is not rapidly dried under elevated temperature conditions (circa 150°C.), Zundel et al. has reported (Physik. Chem. (Frankfurt) 59, 225 [1968]) that the water is hydrogen bonded to three sulfonic acid groups and cannot be removed even under severe drying conditions. However, if drying is carried out at too rapid a rate, the resin beads may be subjected to abrasion and damage with a resultant loss of operating life. Clearly, the rate of drying is critical to an economic process.

The present invention provides apparatus and a method of drying and removing water of condensation during the course of the condensation reaction for maximum efficiency and long (extended) catalyst life.

WO 94/19302 discloses a process for the production of bisphenol-A comprising reacting excess phenol with acetone in a multistage suspended bed reactor column comprising a plurality of perforated trays and downcomers, with catalyst provided on screens associated with the trays. Phenol is charged to above the uppermost tray and portions of acetone are charged to a tray next to the uppermost tray and lower trays. An inert gas is passed upwardly to form a solid liquid suspension and to strip water from the reaction mixture.

The invention comprises a process for the condensation of a monocyclic phenol with a ketone which comprises;
reacting a phenol with a ketone in the presence of a catalytic proportion of an acidic ion-exchange resin, in a reaction zone, whereby a mole of water of condensation is obtained as a co-product of the condensation for each mole of reacted ketone; and
continuously removing the water of condensation from the reaction zone by entrainment in a drying gas passed through the reaction zone.

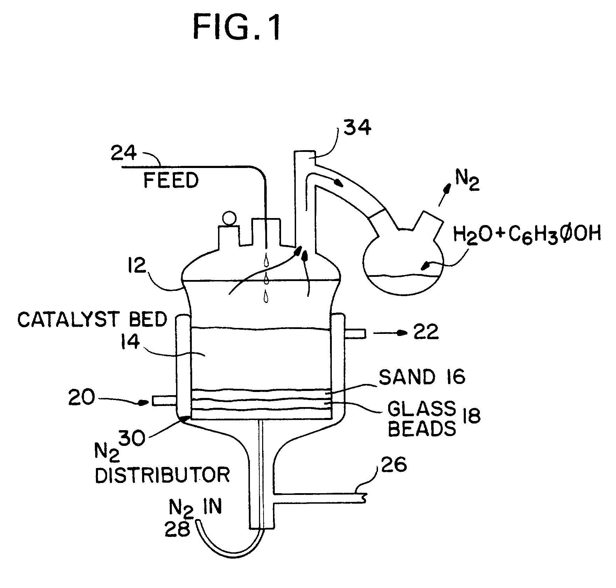

The accompanying drawing is a side view of apparatus, in cross-section, for carrying out the method and process of the invention.

Processes for the synthesis of bisphenols by the condensation of a ketone with a stoichiometric excess of a phenol compound, are generally well known; see for example the descriptions given in the U.S. Patents 3,634,341; 3,760,006; 4,045,379; 4,107,218; 4,294,995; 4,346,247; 4,396,728; 4,400,555; 4,424,283; 4,584,416; and 5,075,511, all of which are incorporated herein by reference thereto. The processes may be carried out continuously or in batch procedures.

In general, the processes may be carried out by bringing the ketone and a stoichiometric excess of the phenol together in a reaction zone, at a temperature of from about 40°C. to 95°C. in the presence of a cationic exchange resin under ambient atmospheric conditions. In some processes, higher than atmospheric pressures may be employed, generally within the range of 101.3-1013 KPa (1-10 atmospheres) The bisphenol product can then be separated from the reaction mixture by conventional procedures well known in the art, including by distillation, crystallization and like methods.

In the commercial process the cationic exchange resin catalyst generally employed is a sulfonated aromatic resin, optionally modified to have mercaptan groups covalently or ionically bound to the resin as promoter sites. Representative of such cationic exchange resins are those described in the U.S. Patents 3,760,006; 3,634,341; 4,045,379; 4,294,995; 4,346,247; and 4,820,740, all of which are incorporated herein by reference thereto.

In general, the resins employed are chemically modified sulfonated aromatic resins. The resins are hydrocarbon polymers having a plurality of pendant sulfonic acid groups. Examples include sulfonated polystyrene or poly(styrenedivinyl-benzene) copolymer and sulfonated phenolformaldehyde resins. The sulfonated resins are commercially available in water swollen form as micro-recticular and macrorecticular types. Specific examples of suitable resins are Amberlite IR-120H, Amberlyst 15H, Amberlyst 31, Dowex 50-X-4, Dowex MSC-1H, Duolite C-26, Permutit QH, Chempro C-2 and Imac C8P/H (Amberlite, Amberlyst, Dowex, Duolite, Permutit, Chempro and Imac are registered U.S. Trademarks). Further examples of such ion exchangers as well as methods for preparing such ion exchangers are described in the Encyclopedia of Polymer Science and Technology, 1967, vol. 7, pages 695 to 708. The exchange capacity of the acidic resin is preferably at least 2.0 meq. H⁺/g of dry resin, with exchange capacities in the range of from 3.0 to 5.5 meq. H⁺/g (dry resin) particularly preferred. Sulfonation may be by the process described in U.S. Patent 2,366,007 which is incorporated herein by reference thereto.

One preferred catalyst used in the process of the present invention is the Amberlyst • gelular types, which are styrene cross-linked with divinylbenzene or like cross-linking monomer and having pendant sulfonic acid groups attached to the aromatic nucleus of the styrene moiety. Most preferred are modified acid ion-exchange resins, wherein the modification comprises esterifying the resin with a mercapto alcohol or by partially neutralizing the resin with an alkyl mercaptoamine. As an alternative to such modification, the bisphenol preparation with the ion-exchange resin may be carried out in the presence of sulfur compound promoters such as alkyl mercaptans, especially 3-mercaptopropionic acid; see for example U.S. Patent 4,191,843 incorporated herein by reference thereto.

The catalyst is employed in a proportion effective for catalytic condensation of the phenol with the ketone. In general, a catalytic proportion is achieved by passage of the mixed reactants through a bed of the resin under conditions for condensation.

The process of the invention is advantageously carried out in the synthesis of a wide variety of bisphenols such as 1-phenyl-1,1-bis(4-hydroxyphenyl) ethane; l-methyl-1,1-bis(4-hydroarylphenyl) ethane and the like. It is a preferred improvement in the synthesis of 2,2-bis-(4-hydroxyphenyl) propane (bisphenol-A).

The commercial preparation of bisphenol-A may be carried out in a number of ways. The reaction zone may comprise a single reactor or two or more reactors in series or in parallel. In the case of a multi-reactor reaction zone, suitably all of the phenol reactant is fed to the first reactor and the acetone reactant is either fed all to the first reactor or divided between the first and second and possibly further reactors.

The molar ratio of phenol to acetone is at least 2 to 1 with a stoichiometric excess of phenol being preferred. Molar ratios are preferably from 3:1 to 40:1, with molar ratios of from 10:1 to 30:1 being most preferred. The optimum ratio depends on reaction conditions, e.g., temperature of reaction and desired conversion.

The reaction temperature in the reactor zone may vary from 40°C. to 95°C. with reaction temperatures in the range of from 55°C. to 90°C. being preferred.

The reaction time in the reactor zone may also vary and depends on reaction temperature and the type of reactor. For example, in a continuous feed, tubular reactor, the liquid hour space velocity (LHSV) of the feed may vary between wide limits with velocities in the range of from 0.2 to 40 liters feedstream/liter catalyst⁻¹/hour⁻¹.

The partially dehydrated catalyst may be charged to an appropriate reactor, for example a fixed bed column reactor and saturated with anhydrous phenol to complete dehydration. Alternatively, the wet ion exchange resin may be charged to the reaction zone and subjected to partial drying as described above in-situ, before saturation with phenol. The phenol addition may be carried out in the reactor or in a different vessel and then transferred to the reactor or reaction zone. The catalyst is further dried by the addition of phenol to the desired level of water content in the catalyst. This level of water will affect the rate of reaction between the phenol and acetone. Essentially about 4 weight percent water in the phenol effluent would bring about a marginally acceptable reaction rate while a level of about 2 weight percent in the effluent or less is preferred.

A level of less than about 1 weight percent is most preferred. The reaction zone is then ready to receive acetone and a molar excess of phenol for condensation following known procedures.

The improved process of the invention will now be described in detail with reference to the accompanying drawing of embodiment apparatus for carrying out the process. As shown in cross-section, a column reactor vessel 12 supports an ion-exchange resin bed 14 on a layer 16 of sand and a layer 18 of glass beads. The vessel 12 is jacketed and warmed by a fluid medium circulated through the jacket between inlet 20 and outlet 22. Mixed reactants enter the vessel 12 through feed inlet 24 and pass by gravity through the catalyst bed 14, which is a reaction zone for condensing the reactants. The product bisphenol drains from the resin bed 14 and is carried from the vessel 12 through conduit 26 for eventual recovery by conventional means. An inert gas, such as nitrogen is inserted into the vessel 12 through inlet 28 and distributed to the resin bed 14 through sparger 30. Bubbles of the drying gas, released from sparger 30 rises and passes through the resin bed 14, counter-current to the reactants and product, continuously entraining and desorbing the co-product water of condensation from the resin bed 14. The gas, with entrained water of condensation is vented from vessel 12 through vent 34 and recycled for re-use by separating the entrained water. The result is the maintenance of a near anhydrous reaction zone environment.

A further benefit results from the counter-current passage of the drying gas through the resin bed, which seems to "agitate" the reaction mixture, improving acetone conversion rates.

The following example describes the manner and process of making and using the invention and sets forth the best mode contemplated by the inventors for carrying out the invention, but should not be construed as limiting.

### EXAMPLE 1

A tubular reactor similar to that shown in the accompanying drawing with a feed inlet and a product outlet was maintained at a temperature of about 65° C. The reactor was charged with a sulfonated polystyrene polymer catalyst prepared in accordance with the procedure described in Example 1 of the U.S. Patent 4,396,728 and having approximately 80% pendant sulfonyl styryl groups and 20% of chemically combined N-propylaminopropyl-mercaptan groups of formula:- 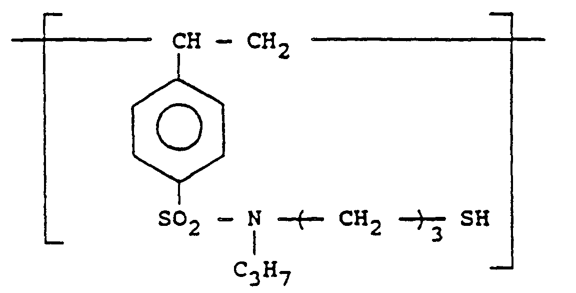 catalyst and fed a reaction mixture of phenol and acetone (6% by weight acetone in pure phenol). At a weighted hour space velocity (WHSV) of 0.5 grams of feed per gram of dry catalyst per hour. During the reaction period, dry nitrogen gas was passed through the catalyst resin bed at a rate of about 2 cc/min./gm. dry catalyst to 20 cc/min./gm. dry catalyst. The gas was vented from the reaction vessel, with entrained water of the condensation reaction in admixture with some phenol and some acetone. The product bisphenol-A was collected over a period of time, with periodic analysis to determine the percentage of conversion and selectivity effected by the catalyst. The analytical results and time of collection is set forth in Table 1, below.

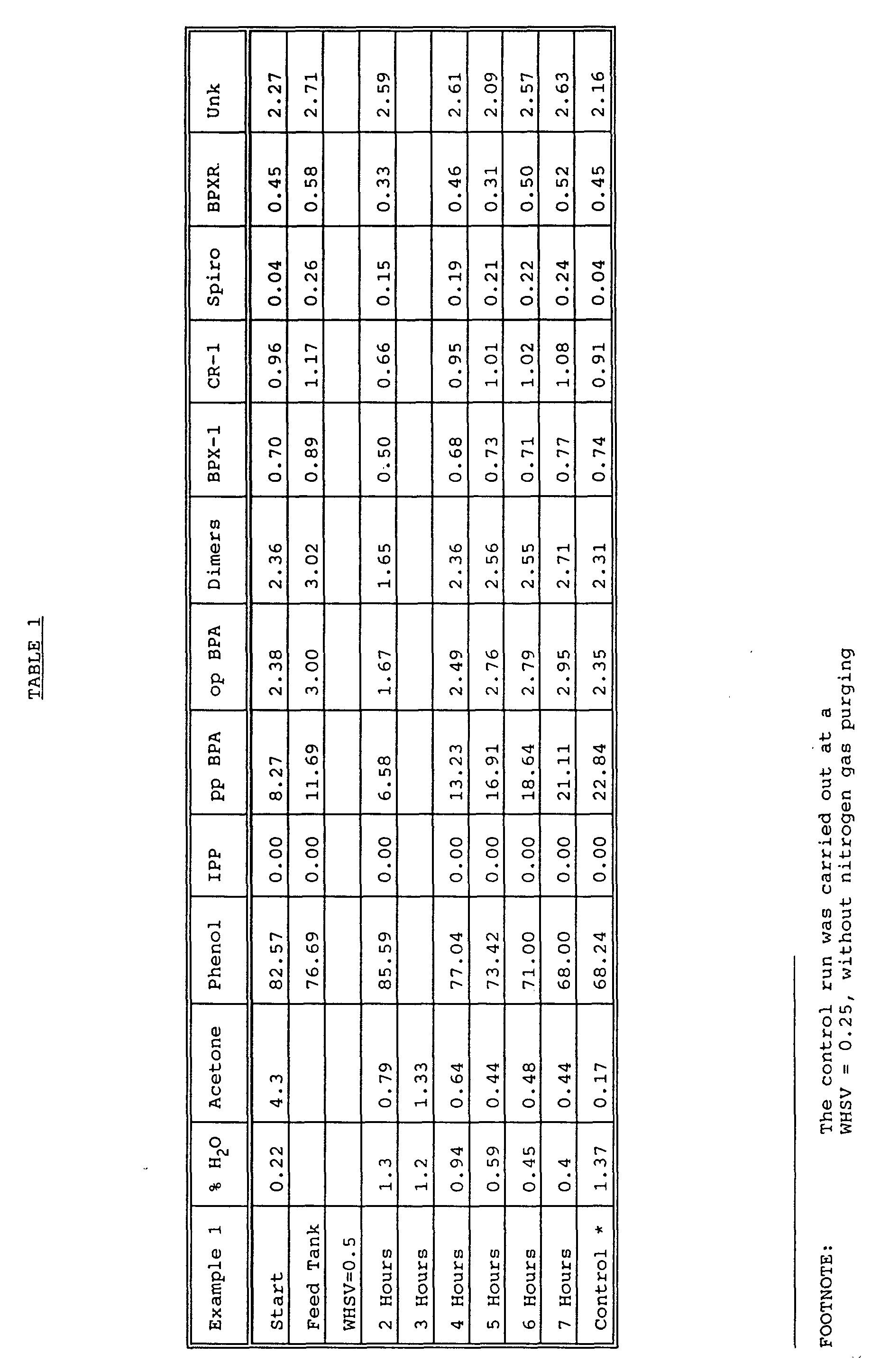

The advantages of the improved method of the invention include the following. The reduction of the co-product water as it is produced avoids retardation of catalyst activity. There is a consequent improved conversion rate, and catalyst life. The improved conversion rates seem to accompany cooler reaction temperatures, which enhances selectivity. There appears to be improved color in the bisphenol product. More importantly 79-80 percent conversion of the acetone occurred at a throughput equal to 4 times that used in standard preparations.

The improved process of the invention has a potential promise of commissioning of new catalyst could be done faster and with much less energy.

### EXAMPLE 2

The general procedure of Example 1, supra., is repeated except that the reaction temperature is 80°C. The test results are shown in Table 2, below.

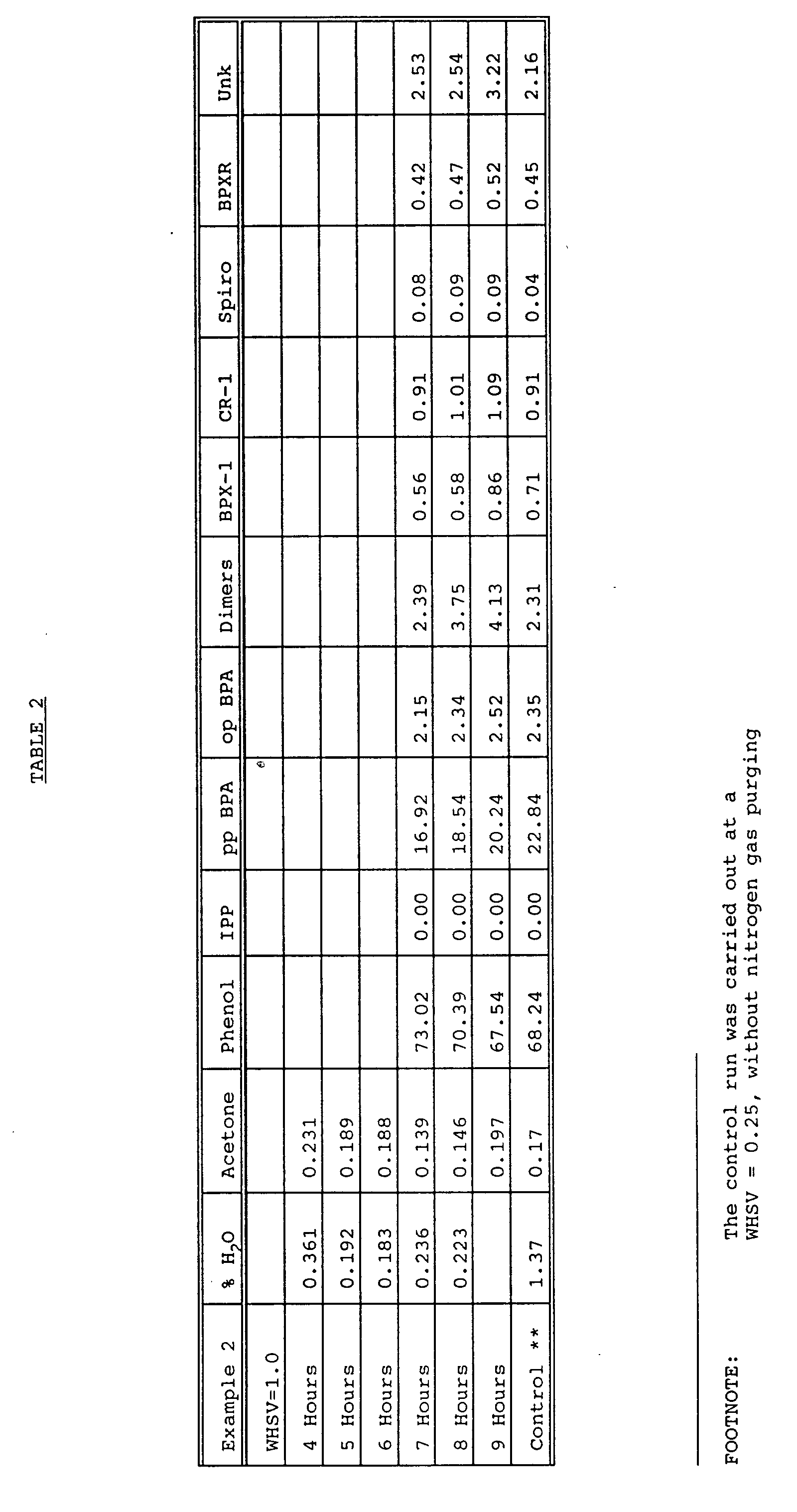

## Claims

1. A process for the condensation of a monocyclic phenol with a ketone, comprising:
reacting a phenol with a ketone in the presence of a catalytic proportion of an acidic ion-exchange resin, in a single resin bed supported by a layer of sand and a layer of glass beads, to form a reaction mixture in a reaction zone, whereby a mole of water of condensation is obtained as a co-product of the condensation for each mole of reacted ketone; and
continuously removing the water of condensation from the reaction zone by entrainment in a drying gas passed through the reaction zone countercurrent to the phenol and the ketone, venting the gas, separating the entrained water from the drying gas and recycling the drying gas for re-use whereby the drying gas agitates the reaction mixture, maintains a near anhydrous reaction zone environment and improves acetone conversion rates.

2. A process of claim 1 wherein the phenol is phenol and the ketone is acetone.

3. A process of claim 1 wherein the drying gas is nitrogen.

4. A process of claim 1 wherein the reaction zone comprises a bed of the resin contained in a reactor.

5. A process for the preparation of bisphenol-A, comprising:
providing a hydrated acidic ion-exchange resin catalyst in a single resin bed supported by a layer of sand and a layer of glass beads, for catalyzing the condensation of phenol with acetone;
vaporizing a portion of the water associated with the hydrated catalyst;
separating sufficient of the water vapor from the catalyst to remove from 25 to 80 percent by weight of said water, whereby a partially dehydrated catalyst is obtained;
contacting the partially dehydrated catalyst with phenol so as to remove additional water from the catalyst until the water content of the effluent phenol contains less than 4 weight percent water;
condensing phenol and acetone in the presence of the dehydrated catalyst, whereby bisphenol-A is formed with water of the condensation; and
passing a drying gas through the dehydrated catalyst to entrain and remove the water of condensation as it is produced venting the gas with the entrained water through a vent separating the entrained water from the gas and recycling the gas for re-use whereby the drying gas agitates the reaction mixture, maintains a near anhydrous reaction zone environment and improves acetone conversion rate.

6. The process of claim 5 wherein the drying gas is nitrogen.

7. The process of claim 6 wherein the nitrogen gas is passed counter-current to the flow of reactants at a rate of from 2 cc to 20 cc/min./gm. dry catalyst.

8. The process of claim 7 carried out at a temperature of from 40° to 95°C.

## Patentansprüche

1. Verfahren zur Kondensation eines monocyclischen Phenols mit einem Keton, umfassend:
Umsetzen eines Phenols mit einem Keton in Gegenwart eines katalytischen Anteils eines sauren Ionenaustauscherharzes in einem einzelnen Harzbett, das durch eine Schicht aus Sand und eine Schicht aus Glasperlen getragen ist, um eine Reaktionsmischung in einer Reaktionszone zu bilden, wobei ein Mol Kondensationswasser als ein Coprodukt der Kondensation für jedes Mol des umgesetzten Ketons erhalten wird und
kontinuierliches Entfernen des Kondensationswassers aus der Reaktionszone durch Mitnehmen in einem trocknenden Gas, das im Gegenstrom zu dem Phenol und dem Keton durch die Reaktionszone strömt, Ablassen des Gases, Abtrennen des mitgenommenen Wassers aus dem trocknenden Gas und Zurückführen des trocknenden Gases zur Wiederverwendung, wobei das trocknende Gas die Reaktionsmischung bewegt und eine nahezu wasserfreie Raktionszonen-Umgebung aufrechterhält und die Aceton-Umwandlungsraten verbessert.

2. Verfahren nach Anspruch 1, worin das Phenol Phenol ist und das Keton Aceton ist.

3. Verfahren nach Anspruch 1, worin das trocknende Gas Stickstoff ist.

4. Verfahren nach Anspruch 1, worin die Reaktionszone ein in einem Reaktor enthaltenes Harzbett umfasst.

5. Verfahren zur Herstellung von Bisphenol-A, umfassend:
Bereitstellen eines hydratisierten sauren Ionenaustauscherharz-Katalysators in einem einzelnen Harzbett, das durch eine Schicht aus Sand und eine Schicht aus Glasperlen getragen ist, um die Kondensation von Phenol mit Aceton zu katalysieren;
Verdampfen eines Teiles des mit dem hydratisierten Katalysator verbundenen Wassers;
Abtrennen von genügend Wasserdampf aus dem Katalysator, um 25 bis 80 Gew.-% des Wassers zu entfernen, wodurch ein teilweise dehydratisierter Katalysator erhalten wird;
in Berührung bringen des teilweise dehydratisierten Katalysators mit Phenol, um zusätzliches Wasser aus dem Katalysator zu entfernen, bis der Wassergehalt des ausströmenden Phenols weniger als 4 Gew.-% Wasser beträgt;
Kondensieren von Phenol und Aceton in Gegenwart des dehydratisierten Katalysators, wodurch Bisphenol-A mit Kondensationswasser gebildet wird, und
Hindurchführen eines trocknenden Gases durch den dehydratisierten Katalysator, um das Kondensationswasser in dem Maße mitzunehmen und zu entfernen, in dem es erzeugt wird,
Ablassen des Gases mit dem mitgenommenen Wasser durch eine Auslassöffnung,
Abtrennen des mitgenommenen Wassers aus dem Gas und Zurückführen des Gases zur Wiederverwendung, wobei das trocknende Gas die Reaktionsmischung bewegt, eine nahezu wasserfreie Reaktionszonen-Umgebung aufrechterhält und die Aceton-Umwandlungsrate verbessert.

6. Verfahren nach Anspruch 5, worin das trocknende Gas Stickstoff ist.

7. Verfahren nach Anspruch 6, worin das trocknende Gas im Gegenstrom zur Strömung der Reaktanten in einer Rate von 2 cm³ bis 20 cm³/min/g des trockenen Katalysators hindurchgeleitet wird.

8. Verfahren nach Anspruch 7, das bei einer Temperatur von 40 bis 95°C ausgeführt wird.

## Revendications

1. Procédé pour la condensation d'un phénol monocyclique avec une cétone, comprenant :
la réaction d'un phénol avec une cétone en présence d'une proportion catalytique d'une résine échangeuse d'ions acide, dans un seul lit de résine supporté par une couche de sable et une couche de billes de verre, pour former un mélange réactionnel dans une zone réactionnelle, une mole d'eau de condensation étant obtenue comme sous-produit de la condensation par mole de cétone ayant réagi ; et
l'élimination en continu de l'eau de condensation de la zone réactionnelle par entraînement dans un gaz de séchage que l'on fait passer dans la zone réactionnelle à contre-courant par rapport au phénol et à la cétone, la purge du gaz, la séparation de l'eau entraînée du gaz de séchage et le recyclage du gaz de séchage pour le réutiliser, le gaz de séchage agitant le mélange réactionnel, maintenant un environnement de la zone réactionnelle presque anhydre et améliorant le taux de conversion de l'acétone.

2. Procédé selon la revendication 1, dans lequel le phénol est le phénol et la cétone est l'acétone.

3. Procédé selon la revendication 1, dans lequel le gaz de séchage est l'azote.

4. Procédé selon la revendication 1, dans lequel la zone réactionnelle comprend un lit de résine contenu dans un réacteur.

5. Procédé pour préparer du bisphénol A, comprenant :
la fourniture d'un catalyseur hydraté de type résine échangeuse d'ions acide dans un seul lit de résine supporté par une couche de sable et une couche de billes de verre, pour catalyser la condensation du phénol avec l'acétone ;
l'évaporation d'une partie de l'eau associée au catalyseur hydraté ;
la séparation de suffisamment de vapeur d'eau du catalyseur pour éliminer 25 à 80 % en poids de ladite eau, un catalyseur partiellement déshydraté étant obtenu ;
la mise du catalyseur partiellement déshydraté en contact avec le phénol de manière à encore éliminer de l'eau du catalyseur jusqu'à ce que l'effluent phénol contienne une teneur en eau inférieure à 4 % en poids ;
la condensation du phénol et de l'acétone en présence du catalyseur déshydraté, le bisphénol A étant formé avec l'eau de la condensation ; et
le passage d'un gaz de séchage à travers le catalyseur déshydraté pour entraîner et éliminer l'eau de condensation lorsqu'elle est produite, la purge du gaz avec l'eau entraînée à travers un évent, la séparation de l'eau entraînée du gaz et le recyclage du gaz pour le réutiliser, le gaz de séchage agitant le mélange réactionnel, maintenant un environnement de la zone réactionnelle presque anhydre et améliorant le taux de conversion de l'acétone.

6. Procédé selon la revendication 5, dans lequel le gaz de séchage est l'azote.

7. Procédé selon la revendication 6, dans lequel on fait passer l'azote gazeux à contre-courant par rapport à l'écoulement des réactifs à un débit de 2 cm³ à 20 cm³/min./g de catalyseur sec.

8. Procédé selon la revendication 7, effectué à une température de 40 à 95 °C.
